# EUROPEAN PATENT APPLICATION

(11) **EP 2 578 255 A1**
(43) Date of publication of application: **10.04.2013**
(21) Application number: 11184083.1
(22) Date of filing: 06.10.2011
(51) Int. Cl.: A61M 5/32

(54) **Needle safety device**

(71) Applicant: Sanofi-Aventis Deutschland GmbH, 65929 Frankfurt (DE)
(72) Inventor: The designation of the inventor has not yet been filed

(57) **Abstract**

Described is a needle safety device (2, 102) comprising a needle hub (2.2, 102.2), a needle (2.3, 102.3) coupled to the needle hub (2.2, 102.2) and having a distal tip (2.3.1, 102.3.1), an outer needle shield (2.1, 102.1) telescopically coupled to the needle hub (2.2, 102.2), and an inner needle shield (2.4, 102.4) telescopically coupled to the outer needle shield (2.1). The needle safety device (2, 102.2) has a first state in which the distal tip (2.3.1, 102.3.1) is covered by the inner needle shield (2.4, 102.4) and the inner needle shield (2.4, 102.4) is movable in a proximal direction relative to the outer needle shield (2.1, 102.1), a second state in which the distal tip (2.3.1, 102.3.1) is exposed from the inner needle shield (2.4, 102.4), and a third state in which the distal tip (2.3.1, 102.3.1) is covered by the inner needle shield (2.4, 102.4) and the inner needle shield (2.4, 102.4) is immovable in the proximal direction relative to the outer needle shield (2.1, 102.1). The needle safety device (2, 102) includes a first locking mechanism (L1, L101) engaging the inner needle shield (2.4, 102.4) to the outer needle shield (2.1, 102.1) in the first state. The needle hub (2.2, 102.2) includes a release element (2.2.1.1, 102.2.2.2) adapted to release the first locking mechanism (L1, L101) in the second state.

## Description

### Background of the Invention

Medicament delivery devices (e.g., pen injectors, syringes, auto-injectors, etc.) that contain a selected dosage of a medicament are well known devices for administering the medicament to a patient. Safety devices for covering a needle of the delivery device before and after use are also well known. Typically, a needle shield of the safety device is either manually moved or automatically to surround the medical needle. Various attempts have been made to develop an optimally sized and functioning safety device. However, there remains a need for an optimal safety needle assembly.

### Summary of the Invention

It is an object of the present invention to provide an improved safety needle assembly that minimizes the risk of an accidental needle stick injury, that is safe to handle, and that provides needle safety before and after the medicament is delivered.

In an exemplary embodiment, a needle safety device according to the present invention comprises a needle hub, a needle coupled to the needle hub and having a distal tip, an outer needle shield telescopically coupled to the needle hub, and an inner needle shield telescopically coupled to the outer needle shield. The needle safety device has a first state in which the distal tip is covered by the inner needle shield and the inner needle shield is movable in a proximal direction relative to the outer needle shield, a second state in which the distal tip is exposed from the inner needle shield, and a third state in which the distal tip is covered by the inner needle shield and the inner needle shield is immovable in the proximal direction relative to the outer needle shield. The needle safety device includes a first locking mechanism engaging the inner needle shield to the outer needle shield in the first state. The needle hub includes a release element adapted to release the first locking mechanism in the second state.

In an exemplary embodiment, the first locking mechanism includes latches on first resilient arms of the outer needle shield adapted to engage recesses on the inner needle shield in the first state. The first arms are deflected by the release element in the second state and disengage the recesses.

In an exemplary embodiment, the first locking mechanism includes latches on first resilient arms of the outer needle shield adapted to abut shoulders on the inner needle shield.

In an exemplary embodiment, the needle safety device further comprises a second locking mechanism engaging the outer needle shield to the needle hub to prevent distal movement of the outer needle shield relative to the needle hub in the first state. The second locking mechanism includes first hooks on the outer needle shield adapted to engage second hooks on the needle hub.

In an exemplary embodiment, the needle safety device further comprises a third locking mechanism engaging the outer needle shield to the needle hub in the second state. The third locking mechanism includes a projection on the needle hub adapted to engage a recess on the outer needle shield.

In an exemplary embodiment, the needle safety device further comprises a fourth locking mechanism engaging the inner needle shield to the outer needle shield in the third state. The fourth locking mechanism includes second resilient arms on the outer needle shield adapted to engage a flange on the inner needle shield.

In an exemplary embodiment, the needle safety device further comprises a spring element adapted to apply a biasing force to the inner needle shield.

In an exemplary embodiment, the needle hub includes a collar adapted to abut the outer needle shield in the second state.

In an exemplary embodiment, the needle safety device further comprises another exemplary embodiment of a third locking mechanism engaging the inner needle shield to the outer needle shield in the third state. The third locking mechanism further comprises a first sub-locking mechanism including a flange on the inner needle shield adapted to engage a recess on the latches. The third locking mechanism further comprises a second sub-Locking mechanism including a finger on the inner needle shield adapted to engage a second resilient arm on the outer needle shield.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given herein below and the accompanying drawings which are given by way of illustration only, and thus, are not limited of the present invention, and wherein:
- Figure 1: shows an isometric view of an exemplary embodiment of a needle safety device mounted to a medicament delivery device.
- Figure 2: shows a distal view of an exemplary embodiment of a needle safety device.
- Figure 3: shows a sectional view of an exemplary embodiment of a needle safety device before use.
- Figure 4: shows a sectional view of an exemplary embodiment of a needle safety device drug use.
- Figure 5: shows a first sectional view of an exemplary embodiment of a needle safety device after use.
- Figure 6: shows a first sectional view of an exemplary embodiment of a needle safety device after use.
- Figure 7: shows an isometric view of another exemplary embodiment of a needle safety device mounted to a medicament delivery device.
- Figure 8: shows a top view of another exemplary embodiment of a needle safety device.
- Figure 9: shows a sectional view of another exemplary embodiment of a needle safety device before use.
- Figure 10: shows a sectional view of another exemplary embodiment of a needle safety device during use.
- Figure 11: shows a first sectional view of another exemplary embodiment of a needle safety device after use.
- Figure 12: shows a second sectional view of another exemplary embodiment of a needle safety device after use.
- Figure 13: shows a second sectional view of another exemplary embodiment of a needle safety device after use.
Corresponding parts are marked with the same reference symbols in all figures.

### Detailed Description

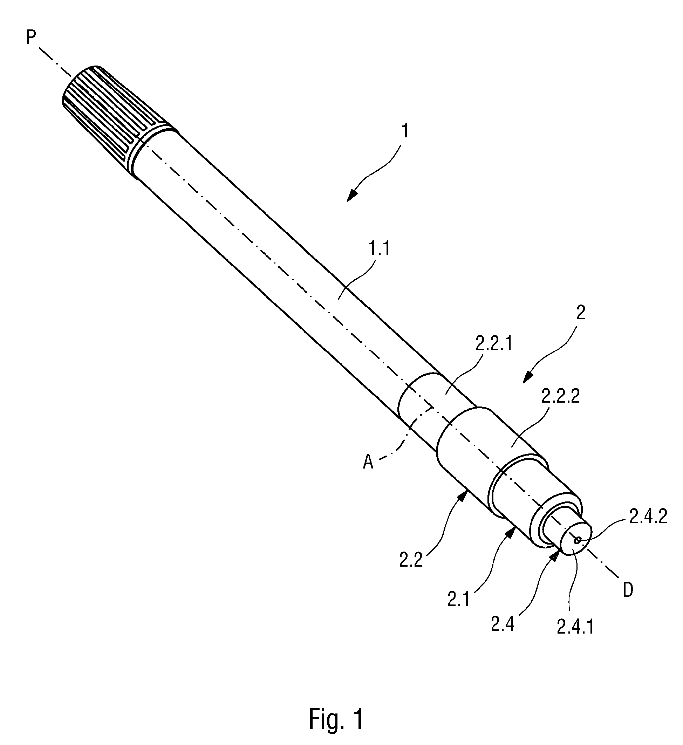

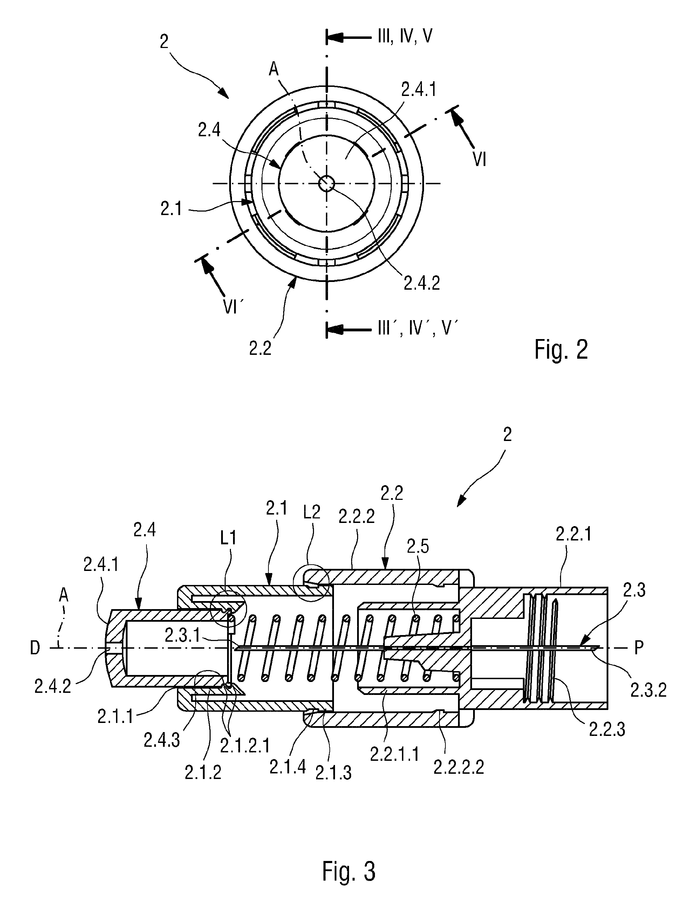

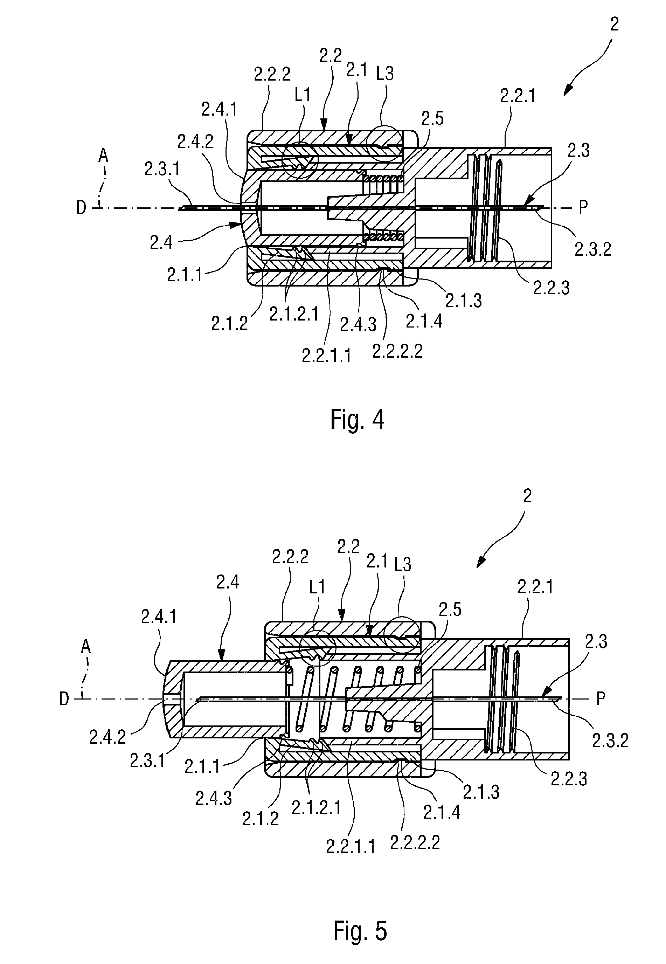

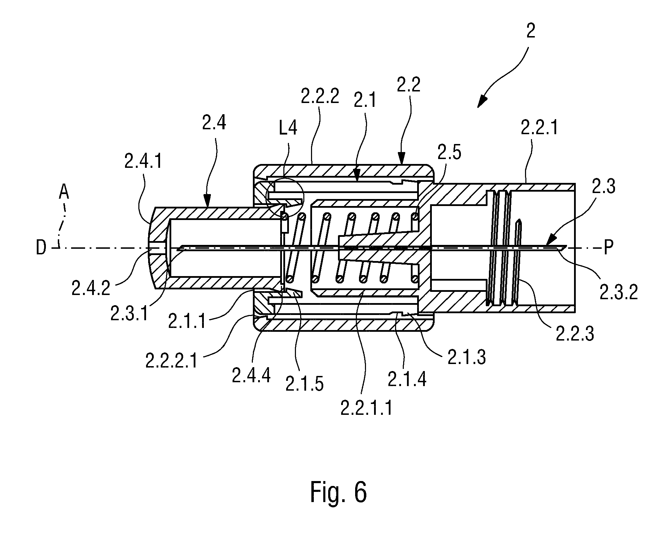

Figure 1 shows an isometric view of an exemplary embodiment of a medicament delivery device 1 with a needle safety device 2. Figure 2 shows a view from a distal direction D of the needle safety device 1 shown in Figure 1 a first plane III-III', IV-IV', V-V' and a second plane VI-VI'. The figures 3 to 6 show sectional views of the exemplary embodiment of the needle safety device 2 of Figure 2, wherein the figure 3 shows the needle safety device 1 before use, figure 4 during use and the figures 5 and 6 after use.

In an exemplary embodiment, the delivery device 1 comprises a housing 1.1 having a longitudinal axis A. In the exemplary embodiment shown, the delivery device 1 is arranged as a pen injector. However, those of skill in the art will understand that the delivery device 1 may be arranged as a syringe, a dental syringe, an auto-injector or a similar device suitable for delivering a medicament to a patient.

In an exemplary embodiment, needle safety device 2 comprises an outer needle shield 2.1 telescopically arranged on a needle hub 2.2 having a needle 2.3 and an inner needle shield 2.4 telescopically arranged on the outer needle shield 2.1. In the exemplary embodiment shown, the inner needle shield 2.4 and the outer needle shield 2.1 are concentrically arranged about the axis A.

In an exemplary embodiment, the needle hub 2.2 comprises a proximal section 2.2.1 adapted to couple to the delivery device 1 and a distal section 2.2.2 adapted to telescopically couple to the outer needle shield 2.1. In the exemplary embodiment shown in Figure 1, the proximal section 2.2.1 has a diameter adapted to engage the distal end of the delivery device 1, and the distal section 2.2.2 has a diameter (which may be larger than the diameter of the proximal section 2.2.1) adapted to engage the outer needle shield 2.1.

Figure 3 shows a section view of the exemplary embodiment of the needle safety device 2 shown in Figure 1 in a first, pre-use state. In the exemplary embodiment shown in Figure 3, in the first state, the outer needle shield 2.1 is in a first axial position (PA1) relative to the needle hub 2.2, and the inner needle shield 2.4 is in a first axial position (PA1 ) relative to the outer needle shield 2.1. In the exemplary embodiment, the proximal section 2.2.1 of the needle hub 2.2 comprises a thread 2.2.3 for mounting the needle safety device 2 to the housing 1.1 of the delivery device 1. In other exemplary embodiments, the needle hub 2.2 may be attached to the housing 1.1 by other suitable couplings, like for example bayonet type couplings, snap-fit couplings, frictional couplings, etc.

The needle 2.3 is coupled to the needle hub 2.2 and includes a pointed distal tip 2.3.1 and a pointed proximal tip 2.3.2. The pointed proximal tip 2.3.2 is adapted to be inserted into a cartridge or container of medicament in the delivery device 1 and the distal tip 2.3.1 is adapted to pierce an injection site. In the first state, the distal tip 2.3.1 of the needle 2.3 is covered by the outer needle shield 2.1 and/or the inner needle shield 2.4.

In an exemplary embodiment, the inner needle shield 2.4 is telescopically coupled to the outer needle shield 2.1 and is adapted to protrude distally from an opening 2.1.1 in the outer needle shield 2.1. A distal face 2.4.1 of the inner shield includes an aperture 2.4.2 for allowing the needle 2.3 to pass through during an injection procedure.

In an exemplary embodiment, a spring element 2.5 is adapted to bias the inner needle shield 2.4 with respect to the needle hub 2.2. The spring element 2.5 may bear distally against the inner needle shield 2.4 and proximally against the needle hub 2.2. The spring element 2.5 may be arranged in an unstressed state in the first state or may be pre-stressed in the first state, providing a biasing force to the inner needle shield 2.4.

As described further below, the needle safety device 2 comprises a plurality of locking mechanisms. In exemplary embodiments, a first locking mechanism L1 engages the inner needle shield 2.4 to the outer needle shield 2.1 and locks the inner needle shield 2.4 against axial movement with respect to the outer needle shield 2.1 in the first state. A second locking mechanism L2 engages the outer needle shield 2.1 to the needle hub 2.2 so that the outer needle shield 2.1 is locked against axial movement in the distal direction D with respect to the needle hub 2.2 in the first state. A third locking mechanism L3 engages the outer needle shield 2.1 to the needle hub 2.2 in a retracted position when the needle safety device 2 is in a second, use state (shown in Figure 4). A fourth locking mechanism L4 engages the inner needle shield 2.4 with the outer needle shield 2.1 and locks the inner needle shield 2.4 against axial movement with respect to the outer needle shield 2.1 in a third, post-use state (shown in figures 5 and 6).

As shown in Figure 3, the needle safety device 2 is in the first state. In the first state, the first locking mechanism L1 prevents axial movement of the inner needle shield 2.4 relative to the outer needle shield 2.1. In an exemplary embodiment, the first locking mechanism L1 includes latches 2.1.2.1 on axial, proximally-directed resilient first arms 2.1.2 on the outer needle shield 2.1 that engage recesses 2.4.3 on the inner needle shield 2.4. In the first state, the second locking mechanism L2 prevents the outer needle shield 2.1 from extending distally relative to the needle hub 2.2 beyond the first axial position (PA1). In an exemplary embodiment, the second locking mechanism L2 includes first hooks 2.1.3 on a proximal end of the outer needle shield 2.1 adapted to engage second hooks 2.2.2.1 (shown in Figure 6) on a distal end of the distal section 2.2.2 of the needle hub 2.2.

Figure 4 shows the exemplary embodiment of the needle safety device 2 in the second state, e.g., when the needle safety device 2 has been pressed against an injection site. In the second state, the outer needle shield 2.1 and the inner needle shield 2.4 are retracted and the distal tip 2.3.1 of the needle 2.3 is exposed through the aperture 2.4.2. In the second state, the outer needle shield 2.1 is in a second, retracted axial position (PA2) relative to the needle hub 2.2. In the second state, the third locking mechanism L3 may prevent distal movement of the outer needle shield 2.1 relative to the needle hub 2.2 when the outer needle shield 2.1 is in the second axial position (PA2). In an exemplary embodiment, the third locking mechanism L3 includes a projection 2.2.2.2 formed on an inner surface of a proximal portion of the distal section 2.2.2 of the needle hub 2.2. The projection 2.2.2.2 may be adapted to engage a recess 2.1.4 on a proximal portion of the outer needle shield 2.1. Further, the outer needle shield 2.1 may be resilient such that it deflects as it passes the projection 2.2.2.2 when the outer needle shield 2.1 is moving proximally relative to the needle hub 2.2, and returns to a non-deflected state when the projection 2.2.2.2 engages the recess 2.1.4.

When the outer needle shield 2.1 is in second axial position (PA2), the first locking mechanism L1 may be released to disengage the outer needle shield 2.1 from the inner needle shield 2.4. In an exemplary embodiment, when the outer needle shield 2.1 is moving proximally relative to the needle hub 2.2 into the second axial position (PA1), the first arms 2.1.2 on the outer needle shield 2.1 engage a release element 2.2.1.1 on the needle hub 2.2 and disengage the recess 2.4.3 on the inner needle shield 2.4. The first arms 2.1.2 may include proximal ramped surfaces to engage a distal ramped surface on the release element 2.2.1.1 which causes the first arms 2.1.2 to deflect radially to release the latches 2.1.2.1 from the recesses 2.4.3. When the inner needle shield 2.4 is disengaged from the outer needle shield 2.4, the inner needle shield 2.4 may move further proximally relative to the needle hub 2.2 into a second axial position (PA2), compressing the spring element 2.5.

Figures 5 and 6 show the exemplary embodiment of the needle safety device 1 in the third state, e.g., when the needle safety device 1 is removed from the injection site. In the third state, the outer needle shield 2.1 remains in the second axial position (PA2), retracted relative to the needle hub 2.2 due to the third locking mechanism L3. However, due to release of the first locking mechanism L1, the inner needle shield 2.4 moves distally relative to the outer needle shield 2.1 into a third axial position (PA3) to cover the distal tip 2.3.1 of the needle 2.3.

In the third state, the fourth locking mechanism L4 prevents axial, proximal movement of the inner needle shield 2.4 relative to the outer needle shield 2.1. In an exemplary embodiment, the fourth locking mechanism L4 includes axial, proximally-directed resilient second arms 2.1.5 that engage a radial flange 2.4.4 on the proximal end of the inner needle shield 2.4. In an exemplary embodiment, the second arms 2.1.5 are formed at 90 degrees about the axis A relative to the first arms 2.1.2 and are shorter than the first arms 2.1.2, so that the second arms 2.1.5 do not engage the release element 2.2.1.1 when the outer needle shield 2.1 is in the second axial position (PA2).

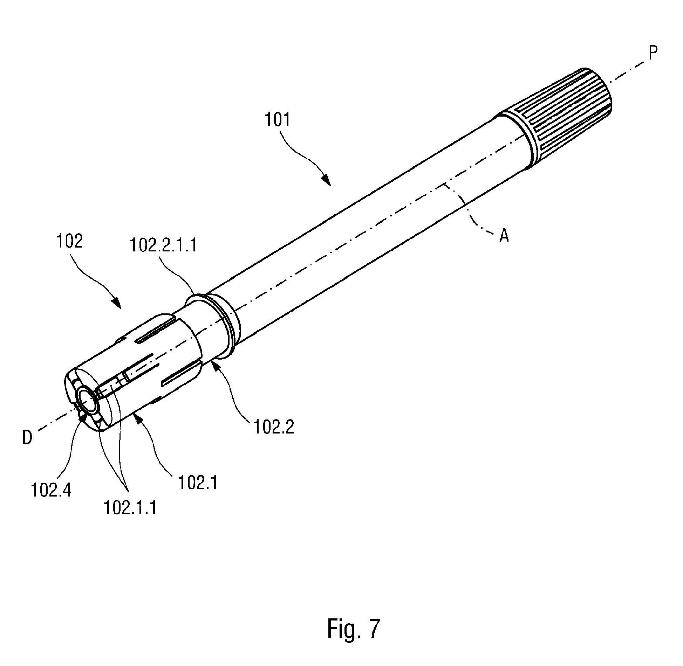

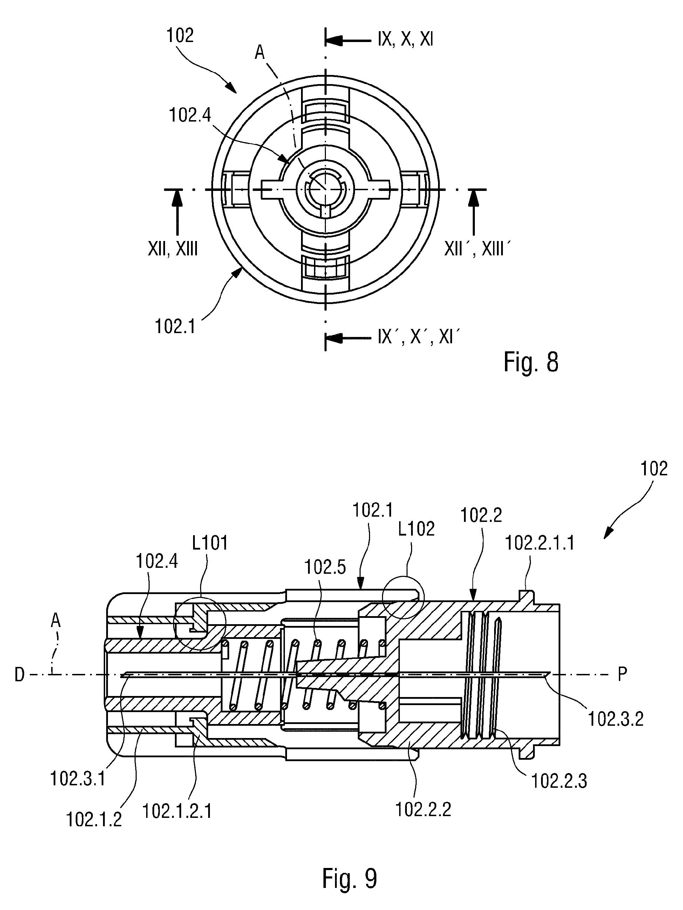

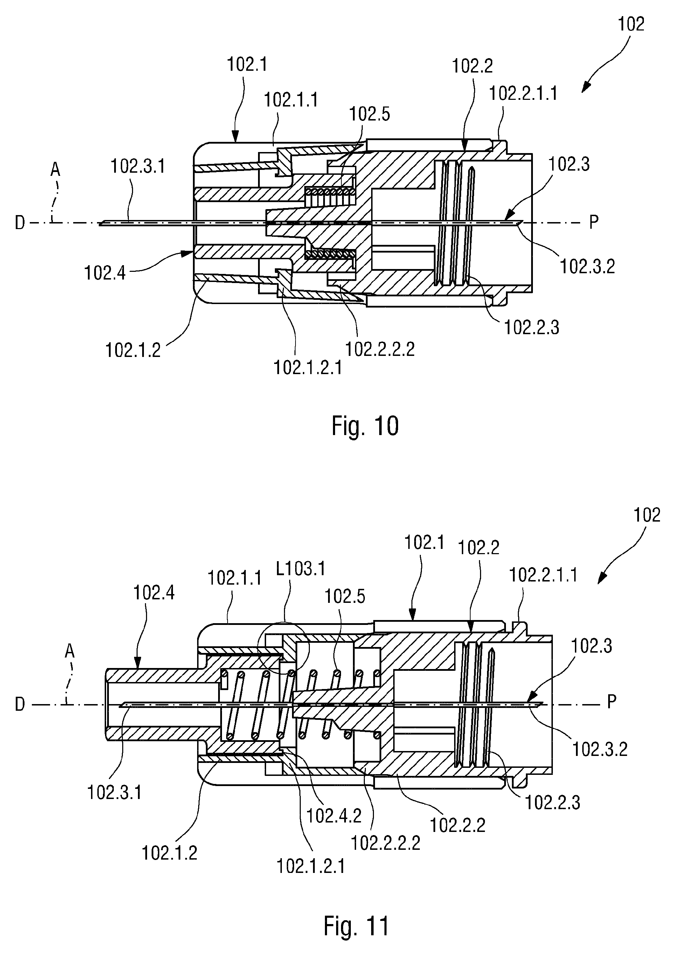

When the needle safety device 2 is removed from the injection site, the inner needle shield 2.4 may translate distally relative to the outer needle shield 2.1 under the force of the spring element 2.5. During distal movement, the flange 2.4.4 may engage the second arms 2.1.5 causing the second arms 2.1.5 to deflect. The second arms 2.1.5 may include a ramped surface to engage a corresponding ramped surface on the flange 2.4.4 which causes the second arms 2.1.5 to deflect radially. When the proximal end of the inner needle shield 2.4 has bypassed the second arms 2.1.5, the second arms 2.1.5 may return to a non-deflected state and engage the proximal end of the inner needle shield 2.4. Thus, when the inner needle shield 2.4 is in the third axial position (PA3), the needle safety device 2 is needle-safe because the inner needle shield 2.4 is prevented from moving proximally relative to the outer needle shield 2.1 by the fourth locking mechanism L4 and the distal tip 2.3.1 of the needle 2.3 remains covered. Figure 7 shows an isometric view of another exemplary embodiment of a medicament delivery device 101 with a needle safety device 102. Figure 2 shows a view from a distal direction D of the needle safety device 102 shown in Figure 7 a first plane XII-XII', XIII-XIII' and a second plane IX-IX', X-X', XI-XI'. The figures 9 to 13 show sectional views of the exemplary embodiment of the needle safety device 102 of Figure 7, wherein the figure 9 shows the needle safety device 102 before use, figure 10 during use and the figures 11 to 13 after use.

In an exemplary embodiment, the delivery device 101 comprises a housing 101.1 having a longitudinal axis A. In the exemplary embodiment shown, the delivery device 101 is arranged as a pen injector. However, those of skill in the art will understand that the delivery device 101 may be arranged as a syringe, a dental syringe, an auto-injector or a similar device suitable for delivering a medicament to a patient.

In an exemplary embodiment, needle safety device 102 comprises an outer needle shield 102.1 telescopically arranged on a needle hub 102.2 having a needle 102.3 and an inner needle shield 102.4 telescopically arranged on the outer needle shield 2.1. In the exemplary embodiment shown, the inner needle shield 102.4 and the outer needle shield 102.1 are concentrically arranged about the axis A. The outer needle shield 102.1 may have one or more axial openings 102.1.1 formed therein.

In an exemplary embodiment, the needle hub 102.2 comprises a proximal section 102.2.1 adapted to couple to the delivery device 101 and a distal section 102.2.2 adapted to telescopically couple to the outer needle shield 102.1. In the exemplary embodiment shown in Figure 7, the proximal section 102.2.1 has a diameter adapted to engage the distal end of the delivery device 101, and the distal section 102.2.2 has a diameter (which may be larger than the diameter of the proximal section 102.2.1) adapted to engage the outer needle shield 102.1.

Figure 9 shows a section view of the exemplary embodiment of the needle safety device 102 shown in Figure 7 in a first, pre-use state. In the exemplary embodiment shown in Figure 9, in the first state, the outer needle shield 102.1 is in a first axial position (PA1) relative to the needle hub 102.2, and the inner needle shield 102.4 is in a first axial position (PA1) relative to the outer needle shield 102.1. In the exemplary embodiment, the proximal section 102.2.1 of the needle hub 102.2 comprises a thread 102.2.3 for mounting the needle safety device 102 to the housing 101.1 of the delivery device 101. In other exemplary embodiments, the needle hub 102.2 may be attached to the housing 101.1 by other suitable couplings, like for example bayonet type couplings, snap-fit couplings, frictional couplings, etc.

The needle 102.3 is coupled to the needle hub 102.2 and includes a pointed distal tip 102.3.1 and a pointed proximal tip 102.3.2. The pointed proximal tip 102.3.2 is adapted to be inserted into a cartridge or container of medicament in the delivery device 101 and the distal tip 102.3.1 is adapted to pierce an injection site. In the first state, the distal tip 102.3.1 of the needle 102.3 is covered by the outer needle shield 102.1 and/or the inner needle shield 102.4.

In an exemplary embodiment, the inner needle shield 102.4 is telescopically coupled to the outer needle shield 102.1. A distal end of the inner needle shield 102.4 includes an aperture for allowing the needle 102.3 to pass through during an injection procedure.

In an exemplary embodiment, a spring element 102.5 is adapted to bias the inner needle shield 102.4 with respect to the needle hub 102.2. The spring element 102.5 may bear distally against the inner needle shield 102.4 and proximally against the needle hub 102.2. The spring element 102.5 may be arranged in an unstressed state in the first state or may be pre-stressed in the first state, providing a biasing force to the inner needle shield 102.4.

As described further below, the needle safety device 102 comprises a plurality of locking mechanisms. In exemplary embodiments, a first locking mechanism L101 engages the inner needle shield 102.4 to the outer needle shield 102.1 and locks the inner needle shield 102.4 against axial movement in the distal direction D with respect to the outer needle shield 102.1 in the first state. A second locking mechanism L102 engages the outer needle shield 102.1 to the needle hub 102.2 so that the outer needle shield 102.1 is locked against axial movement in the distal direction D with respect to the needle hub 102.2 in the first state. A third locking mechanism L103 engages the inner needle shield 102.4 with the outer needle shield 102.1 and locks the inner needle shield 102.4 against axial movement with respect to the outer needle shield 102.1 in a third, post-use state (shown in figures 11 through 13). An optional fourth locking mechanism L104 (not shown) may engage the outer needle shield 102.1 with the needle hub 102.1 in the third, post-use state so that the outer needle shield 102.1 is locked against axial movement in the proximal direction P with respect to the needle hub 102.2

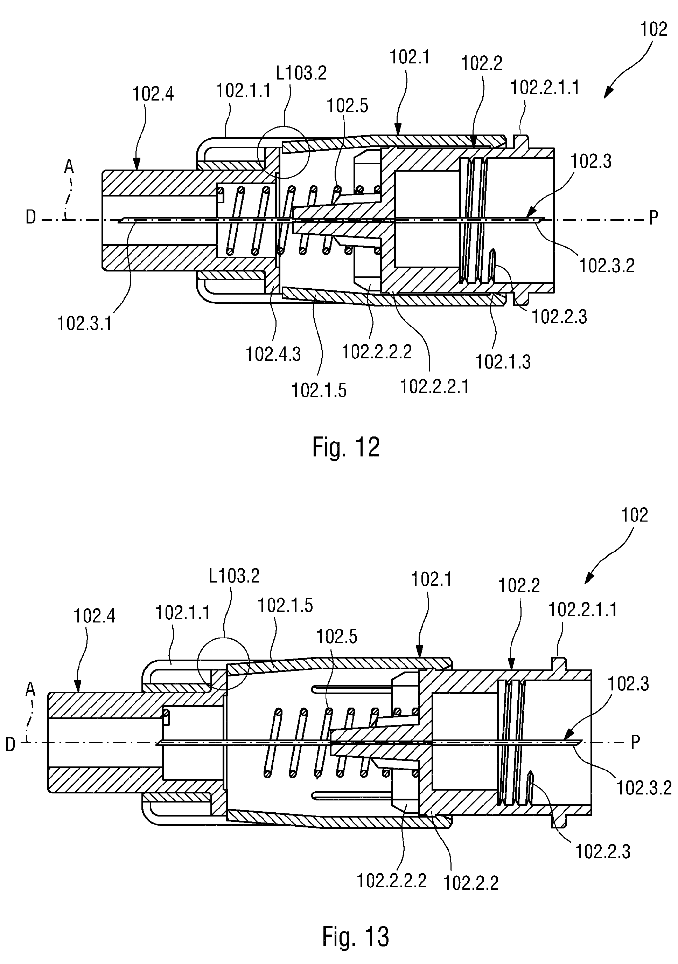

As shown in Figure 9, the needle safety device 1 is in the first state. In the first state, the first locking mechanism L101 prevents axial movement of the inner needle shield 102.4 relative to the outer needle shield 102.1 in the distal direction D. In an exemplary embodiment, the first locking mechanism L1 includes latches 102.1.2.1 on axial, proximally-directed resilient first arms 102.1.2 on the outer needle shield 102.1 adapted to abut shoulders 102.4.1 on the inner needle shield 102.4. The spring element 102.5 may apply a biasing force to the inner needle shield 102.4 so that the shoulders 102.4.1 remain abutting the latches 102.1.2.1 in the first state. In the first state, the second locking mechanism L102 prevents the outer needle shield 102.1 from extending distally relative to the needle hub 102.2 beyond the first axial position (PA1). In an exemplary embodiment, the second locking mechanism L102 includes first hooks 102.1.3 on a proximal end of the outer needle shield 102.1 adapted to engage second hooks 102.2.2.1 (shown in Figure 12) on a distal portion of the distal section 102.2.2 of the needle hub 102.2.

Figure 10 shows the exemplary embodiment of the needle safety device 102 in the second state, e.g., when the needle safety device 102 has been pressed against an injection site. In the second state, the outer needle shield 102.1 and the inner needle shield 102.4 are retracted and the distal tip 102.3.1 of the needle 102.3 is exposed through the aperture of the inner needle shield 102.4. In the second state, the outer needle shield 102.1 and the inner needle shield 102.4 are in a second, retracted axial position (PA2) relative to the needle hub 102.2.

When the outer needle shield 102.1 is in second axial position (PA2), the first locking mechanism L101 may be released to disengage the outer needle shield 102.1 from the inner needle shield 102.4. In an exemplary embodiment, when the outer needle shield 102.1 is moving proximally relative to the needle hub 102.2 into the second axial position (PA1), the first arms 102.1.2 on the outer needle shield 102.1 engage a release element 102.2.2.2 on the needle hub 102.2 and disengage the shoulders on the inner needle shield 102.4. The first arms 102.1.2 may include proximal ramped surfaces 102.1.2.2 adapted to engage a distal ramped surface on the release element 102.2.2.2 which causes the first arms 102.1.2 to deflect radially (e.g., into the openings 102.1.1) to release the latches 102.1.2.1 from the shoulder 102.4.1. When the outer needle shield 102.1 is in the second axial position (PA2), the inner needle shield 102.4 may be in a second axial position (PA2) in which the spring element 102.5 is compressed.

As shown in the exemplary embodiment in Figure 10, the proximal section 102.2.1 of the needle hub 102.2 may include a collar 102.2.1.1 adapted to abut a proximal end of the outer needle shield 102.1. The collar 102.2.1.1 may limit proximal movement of the outer needle shield 102.1 relative to the needle hub 102.2 in the second axial position (PA2).

Figures 11, 12 and 13 show the exemplary embodiment of the needle safety device 102 in the third state, e.g., when the needle safety device 102 is removed from the injection site. In the third state, due to release of the first locking mechanism L101, the inner needle shield 102.4 is capable of moving distally relative to the outer needle shield 102.1 into a third axial position (PA3) to cover the distal tip 102.3.1 of the needle 102.3.

In the third state, the third locking mechanism L103 prevents axial, proximal movement of the inner needle shield 102.4 relative to the outer needle shield 102.1. In an exemplary embodiment, the third locking mechanism L103 includes a first sub-mechanism L103.1 including recess 102.1.2.1.1 formed on a distal portion of the latches 102.1.2.1 that is adapted to engage a flange 102.4.4 on the proximal end of the inner needle shield 102.4.

When the needle safety device 102 is removed from the injection site, the inner needle shield 102.4 may translate distally relative to the outer needle shield 102.1 under the force of the spring element 102.5. During distal movement, the inner needle shield 102.4 may abut the first arms 102.1.2 to drive the outer needle shield 102.1 distally relative to the needle hub 102.2. When the first arms 102.1.2 have bypassed the release element 102.2.1.1, the first arms 102.1.2 return to a non-deflected state, and the recess 102.1.2.1.1 formed on the distal portion of the latches 102.1.2.1 is aligned with the flange 102.4.2 on the proximal end of the inner needle shield 102.4 to prevent proximal movement of the inner needle shield 102.4 relative to the outer needle shield 102.1. Thus, when the inner needle shield 102.4 is in the third axial position (PA3), the needle safety device 102 is needle-safe because the inner needle shield 102.4 is prevented from moving proximally relative to the outer needle shield 102.1 and the distal tip 2.3.1 of the needle 2.3 remains covered.

As shown in Figure 12, the third locking mechanism L103 may also include a second sub-mechanism L103.2 including a finger 102.4.3 formed on a proximal end of the inner needle shield 102.4 and a second resilient arm 102.1.5, biased radially toward the axis A, formed on the outer needle shield 102.1. In an exemplary embodiment, the openings 102.1.1 and the second arms 102.1.5 are formed at 90 degrees from each other on the outer needle shield 102.1. When the needle safety device 102 is removed from the injection site, the inner needle shield 102.4 may translate distally relative to the outer needle shield 102.1 under the force of the spring element 102.5. During distal movement, the finger 102.4.3 on the inner needle shield 102.4 may abut the second arms 102.1.5 on the outer needle shield 102.1, causing the second arms 102.1.5 to deflect radially. When the fingers 102.4.3 have bypassed the second arms 102.1.5, the second arms 102.1.5 return to a non-deflected state, and the fingers 102.4.3 engage the outer needle shield 102.1. In the non-deflected state, the second arms 102.1.5 prevent proximal movement of the inner needle shield 102.4 relative to the outer needle shield 102.1. Thus, when the inner needle shield 102.4 is in the third axial position (PA3), the needle safety device 102 is needle-safe because the inner needle shield 102.4 is prevented from moving proximally relative to the outer needle shield 102.1 and the distal tip 2.3.1 of the needle 2.3 remains covered.

Those of skill in the art will understand that in another exemplary embodiment, the finger 102.4.3 may be resilient and the second arms 102.1.5 may be rigid.

Figure 13 shows an exemplary embodiment of the needle safety device 102 in which the spring element 102.5 has expanded and forced the outer needle shield 102.1 into a third axial position (PA3) relative to the needle hub 102.2.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A needle safety device (2, 102), comprising:
a needle hub (2.2, 102.2);
a needle (2.3, 102.3) coupled to the needle hub (2.2, 102.2), the needle (2.3, 102.3) having a distal tip (2.3.1, 102.3.1);
an outer needle shield (2.1, 102.1) telescopically coupled to the needle hub (2.2, 102.2); and
an inner needle shield (2.4, 102.4) telescopically coupled to the outer needle shield (2.1),
wherein the needle safety device (2, 102.2) has a first state in which the distal tip (2.3.1, 102.3.1) is covered by the inner needle shield (2.4, 102.4) and the inner needle shield (2.4, 102.4) is movable in a proximal direction relative to the outer needle shield (2.1, 102.1), a second state in which the distal tip (2.3.1, 102.3.1) is exposed from the inner needle shield (2.4, 102.4), and a third state in which the distal tip (2.3.1, 102.3.1) is covered by the inner needle shield (2.4, 102.4) and the inner needle shield (2.4, 102.4) is immovable in the proximal direction relative to the outer needle shield (2.1, 102.1),
wherein the needle safety device (2, 102) includes a first locking mechanism (L1, 7 L101) engaging the inner needle shield (2.4, 102.4) to the outer needle shield (2.1, 102.1) in the first state, and
wherein the needle hub (2.2, 102.2) includes a release element (2.2.1.1, 102.2.2.2) adapted to release the first locking mechanism (L1, L101) in the second state.

2. The needle safety device according to claim 1, wherein the first locking mechanism (L1) includes latches (2.1.2.1) on first resilient arms (2.1.2) of the outer needle shield (2.1) adapted to engage recesses (2.4.3) on the inner needle shield (2.4) in the first state.

3. The needle safety device according to claim 2, wherein the first arms (2.1.2) are deflected by the release element (2.2.1.1) in the second state and disengage the recesses (2.4.3).

4. The needle safety device according to any of the preceding claims, wherein the first locking mechanism (L101) includes latches (102.1.2.1) on first resilient arms (102.1.2) of the outer needle shield (102.1) adapted to abut shoulders (102.4.1) on the inner needle shield (102.4).

5. The needle safety device according to any of the preceding claims, further comprising:
a second locking mechanism (L2, L102) engaging the outer needle shield (2.1, 102.1) to the needle hub (2.2, 102.2) to prevent distal movement of the outer needle shield (2.1, 102.1) relative to the needle hub (2.2, 102.2) in the first state.

6. The needle safety device according to claim 5, wherein the second locking mechanism (L2, L102) includes first hooks (2.1.3, 102.1.3) on the outer needle shield (2.1, 102.1) adapted to engage second hooks (2.2.2.1, 102.2.2.1) on the needle hub (2.2, 102.2).

7. The needle safety device according to claims 5 or 6, further comprising:
a third locking mechanism (L3) engaging the outer needle shield (2.1) to the needle hub (2.2) in the second state.

8. The needle safety device according to claim 7, wherein the third locking mechanism (L3) includes a projection (2.2.2.2) on the needle hub (2.2) adapted to engage a recess (2.1.4) on the outer needle shield (2.1).

9. The needle safety device according to claims 7 or 8, further comprising:
a fourth locking mechanism (L4) engaging the inner needle shield (2.4) to the outer needle shield (2.1) in the third state.

10. The needle safety device according to claim 9, wherein the fourth locking mechanism (L4) includes second resilient arms (2.1.5) on the outer needle shield (2.1) adapted to engage a flange (2.4.4) on the inner needle shield (2.4).

11. The needle safety device according to any of the preceding claims, further comprising:
a spring element (2.5) adapted to apply a biasing force to the inner needle shield (2.4).

12. The needle safety device according to any of the preceding claims, wherein the needle hub (102.2) includes a collar (102.2.1.1) adapted to abut the outer needle shield (102.1) in the second state.

13. The needle safety device according to claims 5 or 6, further comprising:
a third locking mechanism (L103) engaging the inner needle shield (102.4) to the outer needle shield (102.1) in the third state.

14. The needle safety device according to claim 13, wherein the third locking mechanism (L103) further comprises:
a first sub-locking mechanism (L103.1) including a flange (102.4.4) on the inner needle shield (102.4) adapted to engage a recess (102.1.2.1.1) on the latches (102.1.2.1).

15. The needle safety device according to claim 14, wherein the third locking mechanism (L103) further comprises:
a second sub-Locking mechanism (L103.2) including a finger (102.4.3) on the inner needle shield (102.4) adapted to engage a second resilient arm (102.1.5) on the outer needle shield (102.1).
